Europäisches Patentamt

European Patent Office

Office européen des brevets

(11) Veröffentlichungsnummer: **0 325 966**
**A2**

(12) **EUROPÄISCHE PATENTANMELDUNG**

(21) Anmeldenummer: **89100570.4**

(22) Anmeldetag: **13.01.89**

(51) Int. Cl.⁴: **C11D 17/04**

(30) Priorität: 27.01.88 DE 8800910 U
15.02.88 DE 8801919 U
12.10.88 DE 3834699

(43) Veröffentlichungstag der Anmeldung:
**02.08.89 Patentblatt 89/31**

(84) Benannte Vertragsstaaten:
**ES GR**

(71) Anmelder: **Henkel Kommanditgesellschaft auf Aktien**
**Postfach 1100 Henkelstrasse 67**
**D-4000 Düsseldorf 1(DE)**

(72) Erfinder: **Weber, Rudolf**
**Am Nettchesfeld 51**
**D-4000 Düsseldorf 13(DE)**
Erfinder: **Mehlhorn, Lutz**
**Schroersdyk 20**
**D-4150 Krefeld(DE)**
Erfinder: **Thiel, Daniele, Dr.**
**Urdenbacher Dorfstrasse 26**
**D-4000 Düsseldorf(DE)**
Erfinder: **Janschuk, Theodor**
**Friedingsstrasse 54**
**D-4000 Düsseldorf 12(DE)**

(74) Vertreter: **Rieder, Hans-Joachim, Dr. et al**
**Corneliusstrasse 45 Postfach 11 04 51**
**D-5600 Wuppertal 11(DE)**

(54) **Faservlies.**

(57) Die Erfindung schlägt zur Verbesserung des Waschergebnisses vor, ein Faservlies (1) als Schmutzfänger in der Waschlauge zu verwenden. Insbesondere gelangen hierzu Faservliese zum Einsatz, die aus einer Kombination unterschiedlicher Kunststoffasern bestehen. Bevorzugt werden Polypropylen-und/oder Polyamid- und/oder kationisierte Zellulosefasern.

FIG. 1

## Faservlies

Die Erfindung betrifft ein Faservlies.

Bekannterweise verliert bei einem Waschprozeß die Lauge ihre Reinigungswirkung mit der Zunahme an Schmutzteilen. Dies kann bis zu einer Übersättigung der Lauge an Schmutzteilen führen, so daß keine Reinigungswirkung mehr auftritt. Die Lauge ist somit nicht mehr in der Lage, weitere Schmutzteile in der Schwebe zu halten. Daher wird insbesondere stark verschmutzte Wäsche einer Vor- und Hauptwäsche ausgesetzt.

Der Erfingung liegt die Aufgabe zugrunde, in einfacher Weise den Waschprozeß zu verbessern.

Gelöst wird diese Aufgabe durch die Zugabe eines Faservlieses. welches als Schmutzfänger in die Waschlauge oder dergleichen eingebracht wird. Vorzugsweise weist das Faservlies Fasern aus Kunststoff auf. Die Anordnung läßt sich den jeweiligen Erfordernissen anpassen, wenn eine Kombination verschiedener Fasern eingesetzt wird, das heißt Fasern aus unterschiedlichen Materialien.

Das erfindungsgemäße, den Schmutzfängereffekt aufweisende Faservlies ist vorzugsweise durch eine geeignete Kombination verschiedener Fasern gekennzeichnet, welche beispielsweise bei einer in einer Waschmaschine befindlichen Waschlauge die darin enthaltenen fettigen und pigmenthaltigen Verschmutzun gen bzw. Farbstoffpartikel gierig aufnehmen. Demgemäß wird die Waschlauge entlastet. wodurch sie über einen längeren Zeitraum ihre Waschkraft behält. Hieraus resultiert gegenüber einer vergleichbaren Dosierung bei einem Waschprozeß eine vergrößerte Reinigungswirkung der zu waschenden Textilien, während die Verschmutzungen und Belastungen zu einer Vergrauung bzw. Verfärbung des beigefügten Faservlieses führen. Anhand des sichtbaren Verschmutzungsgrades, was einem Indikatoreffekt entspricht, erkennt der Verbraucher die Wirksamkeit des Faservlieses. Aufgrund der Schmutzfängereigenschaft kann auch mit einer geringeren Menge an Waschmittel gearbeitet werden unter Verringerung einer Belastung des Abwasserkanalnetzes.

Eine besonders gute Bindung von Schmutzteilchen läßt sich dabei durch die Kombination unterschiedlicher Kunststoffasern erreichen. In Versuchen haben sich insbesondere Polypropylen- und/oder Polyamid- und/oder kationisierte Zellulosefasern als günstig erwiesen. Ein derart gestaltetes Faservlies zeichnet sich durch eine große Haltbarkeit und durch ein hohes Schmutzfassungsvermögen aus. Durch eine solches Faservlies werden aus der Waschlauge die das Schmutztragevermögen belastenden abgelösten Schmutzsubstanzen in erheblichem Maße entfernt.

Versuche haben gezeigt, daß Polypropylenfasern, insbesondere Fett binden und einer Vergrauung entgegenwirken. Insofern wird eine Art Pigmentschutz der Wäschestücke realisiert. Polyamidfasern hingegen binden in der Waschlauge enthaltene Farbstoffe. Kationische Polymere, insbesondere kationisierte Zellulosefasern wiederum binden vorzugsweise Aufheller und auch Farbstoffe. Man stelle sich vor, daß ein farbiges Wäschestück mit einer Waschlauge behandelt wurde, die Aufheller enthielt. Die Folge ist, daß nach der Wäsche das Wäschestück nicht mehr die Original-Farbe aufweist, sondern einen helleren Farbton angenommen hat. In einer nachfolgenden Wäsche gibt ein derartiges Wäschestück die an ihm verbliebe nen Aufhellerreste wieder an die Waschlauge ab, so daß selbst dann, wenn nunmehr ein aufhellerfreies Waschmittel eingesetzt wird, Aufhellerreste die Waschlauge belasten, obwohl hier z. B. ein derartiger Aufheller gar nicht erwünscht ist. Wird nunmehr das erfindungsgemäße Faservlies aus kationischen Polymeren eingesetzt, so werden die Aufhellerreste gebunden und können demzufolge sich nicht nachteilig auf die Wäsche auswirken.

Der Einsatzbereich des Faservlieses Läßt sich dadurch vergrößern, daß es einseitig eine flüssigkeitsundurchlässige Folie aufweist. Dann kann dieses Faservlies sogar für die Dosierung eines flüssigen Wäschebehandlungsmittels, insbesondere Flüssig-Waschmittels dienen, indem dieses aus einer Vorratsflasche oder einem Dosierbehälter heraus auf die Zellstruktur des Faservlieses gegossen wird. Bei diesem Vorgang wird das Faservlies mit seiner Folie nach unten vorzugsweise auf eine ebene Unterlage gelegt. Durch die Saugwirkung des Faservlieses wird die portionierte Wäschebehandlungsflüssigkeit aufgenommen und "gebunden". Die flüssigkeitsundurchlässige Folie verhindert, daß die Wäschebehandlungsflüssigkeit durch die Zellstruktur hindurchtritt und somit teilweise verlorengeht bzw. die Unterlage benetzt. Die Bindungswirkung einer entsprechenden Zellstruktur ist so groß, daß nach der Verteilung des flüssigen Wäschebehandlungsmittels auch dann kein Herauslaufen des Mittels erfolgt, wenn die Faserstruktur zum Einbringen in die Trommel der Waschmaschine senkrecht gehalten wird. Das so getränkte Faservlies wird für den Waschvorgang zwischen die zu behandelnden Textilien gelegt. Die Saugwirkung des Faservlieses verhindert, daß Wäschebehandlungsmittelanteile durch Einlaufen in den Laugenstutzen der Waschmaschine verlorengehen. Dadurch erfüllt das Faservlies eine Doppelfunktion: einerseits dient es zur Dosierung von Waschmittel und andererseits als Schmutzfänger. Da es sich bezüglich des einen Dosierspeicher bildenden Faservlieses um eine saugfähige, weiche Zellstruktur handelt, ist zum einen eine Geräuschentwicklung vermieden und zum anderen eine textilfreundliches Verhalten gewährleistet. Auch ist gewährleistet, daß die Übertragung des Wäschebehandlungsmittels an die zu behandelnden Textilien beim Waschvorgang im wesentlichen erst dann erfolgt, wenn Wasser hinzutritt. Als flüssigkeitsundurchlässige Folie eignet sich insbesondere flexible Kunststoffolie. Es ist möglich, Faservlies und Folie getrennt zu fertigen und dann miteinander zu verbinden. Die Verbindung kann durch Schweißen, durch Verkleben oder durch Vernähen geschaffen werden. Entweder kann die Verbindung ganzflächig oder punktuell erfolgen. Möglich ist auch eine randseitige Verbindung. Der Aufbau des Faservlieses aus den in den Ansprüchen genannten Fasern bewirkt eine ausgezeichnete Bindung des Flüssigwaschmittels, so daß ein Herauslaufen der Flüssigkeit beim Hantieren des Faservlieses nicht zu befürchten ist. Die Speicherfähigkeit ist derart groß gewählt (durch Einstellung von Vliesgröße, Vlieshöhe und Wahl der Fasern), daß die für einen Waschprozeß erforderliche Flüssigwaschmittelmenge problemlos gespeichert werden kann. Besonders geeignet sind Faservliese aus den bereits genannten Polypropylen- und/oder Polyamid- und/oder kationisierten Zellulosefasern.

Ferner ist die Erfindung durch ein Verfahren gemäß Anspruch 10 gekennzeichnet.

Ferner ist die Verwendung des mit Folie versehenen Faservlieses zum vor dem Waschprozeß erfolgenden Detachieren von Verschmutzungen an Textilien möglich. Das Faservlies kann verschiedene Formen besitzen. Es kann als Tuch unterschiedlichen Grundrisses, als Beutel, als Waschhandschuh etc. gestaltet sein. Bei einer solchen örtlichen Detachur wird das Flüssig-Waschmittel auf das Faservlies aufgebracht und dann auf den Fleck bzw. die Verschmutzung abgedrückt. Die Übertragung erfolgt durch mechanisches Einarbeiten, wobei die Folienseite der Hand zugekehrt ist. Damit wird erreicht, daß die waschaktiven Substanzen gezielt auf die verfleckten Stellen übertragen werden. Im übrigen erfolgt kein Produktkontakt mit der Haut. Um ein Durchdrücken des zur Detaschur benutzten Mittels zu verhindern (Benetzung der Tischplatte usw), wird ein Teil des mit Folie versehenen Vliestuches unter die Fleckstelle gelegt. Anschließend kann das Faservlies dem Waschvorgang beigegeben werden, so daß das Behandlungsmittel vollständig wirksam wird.

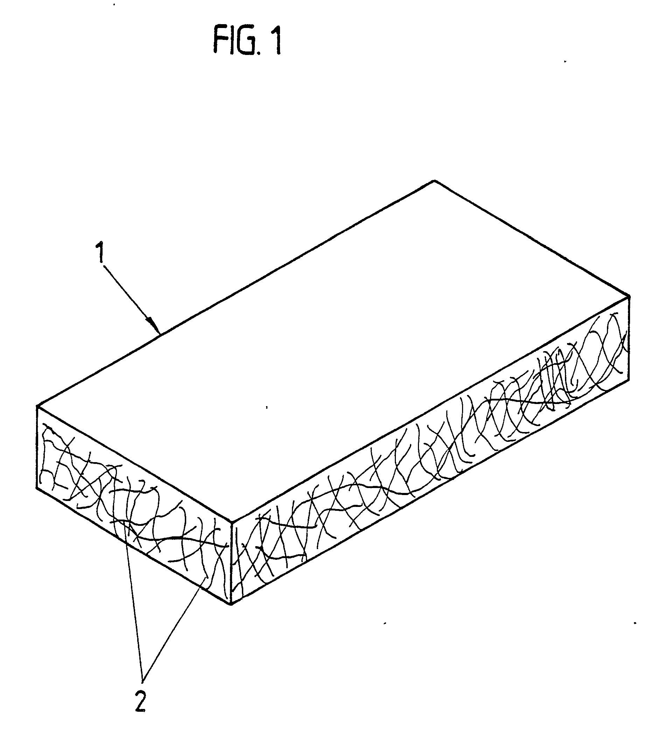

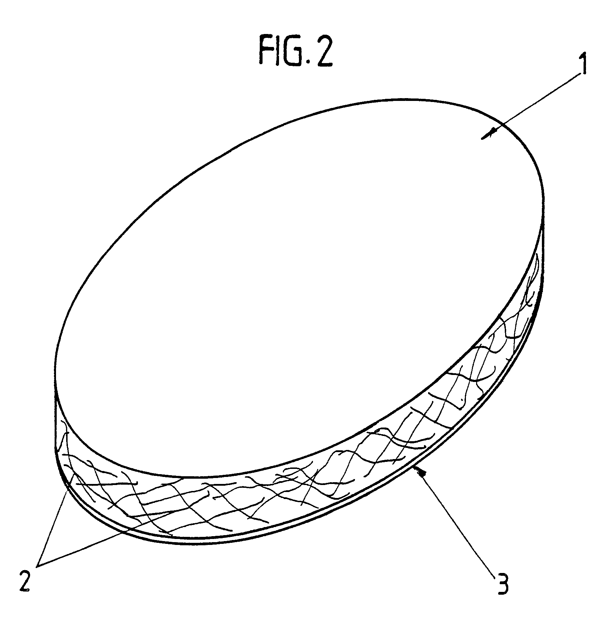

Nachstehend werden zwei Ausführungsbeispiele der Erfindung anhand der Figuren 1 und 2 erläutert. Es zeigt:

Fig. 1 eine in Rechteckform erstelltes Faservlies in per spektivischer Darstellung gemäß dem erstem Ausführungsbeispiel,

Fig. 2 die zweite Ausführungsform eines kreisförmig gestalteten Faservlieses, welches einseitig mit einer flüssigkeitsundurchlässigen Folie versehen ist.

Figur 1 zeigt ein Faservlies 1 rechteckigen Grundrisses. Dieses besteht aus einer Kombination unterschiedlicher Kunststoffasern. Bezüglich derselben handelt es sich um Polypropylen- und Polyamidfasern 2.

Dieses derart gestaltete Faservlies wird mit den zu waschenden Textilien in die Waschmaschinentrommel eingebracht. Zufolge des Schmutzfängereffektes werden beim Durchfluten des Faservlieses von den Kunststoffasern Schmutzteile angezogen und gebunden unter Entlastung der Waschlauge, so daß deren Wirkkraft über einen längeren Zeitraum erhalten bleibt.

Das Faservlies 1 ist mehrmals einsetzbar. Der Verschmutzungsgrad des Faservlieses zeigt an, ob eine Erneuerung notwendig ist.

Gemäß der in Fig. 2 veranschaulichten Ausgestaltung besitzt das Faservlies 1 eine Kreisform. An der einen Seite ist dasselbe mit einer flüssigkeitsundurchlässigen Folie 3 versehen. Diese Maßnahme erlaubt daher die Aufnahme von Wäschebehandlungsflüssigkeit. Zu diesem Zweck wird das Faservlies mit seiner Folie 3 nach unten auf eine vorzugsweise ebene Unterlage gelegt, und dann wird zentral flüssiges Wäschebehandlungsmittel, insbesondere Flüssigwaschmittel, auf das Faservlies 1 gegossen, wodurch aufgrund der Saugwirkung des Faservlieses 1 eine Bindungswirkung eintritt. Ein Durchtritt des flüssigen Wäschebehandlungsmittels durch das Faservlies 2 wird insbesondere im Bereich anfänglich hoher Flüssigkeitskonzentrationen (z. B. im Gießkegelbereich) durch die Folie 3 verhindert. Ist der Dosiervorgang beendet, so wird sich aufgrund der Kapillarwirkung des Faservlieses eine im wesentlichen gleichmäßige Flüssigkeitskonzentration über die benetzte Vliesfläche einstellen.

Dieses derart getränkte Faservlies wird dem Waschprozeß zugegeben, wobei es das Waschmittel an die Lauge abgibt. Gleichzeitig bzw. verzögert erfüllt das Faservlies dann die Aufgabe eines Schmutzfängers.

Zwecks einer Vorbehandlung von Flecken bzw. starken Verschmutzungen an waschbaren Textilien ist es möglich, auf dem Faservlies befindliches Flüssig-Waschmittel auf die entsprechenden Stellen aufzutragen. Die Übertragung erfolgt dabei durch mechanisches Einarbeiten, wobei die Folienseite der Hand zugekehrt ist. Anschließend kann das Faservlies ebenfalls der Wäsche beigegeben werden, so daß beim anschließenden Waschprozeß das restliche Auswaschen des Waschmittels geschieht verbunden mit dem Schmutzfängereffekt.

unter Entlastung der Waschlauge von entsprechenden Schmutzarten wie fettige und pigmenthaltige Verschmutzungen bzw. Farbstoffpartikel.

. Alle in der Beschreibung erwähnten und in der Zeichnung dargestellten neuen Merkmale sind erfindungswesentlich, auch soweit sie in den Ansprüchen nicht ausdrücklich beansprucht sind.

**Ansprüche**

1. Faservlies, gekennzeichnet zur Verwendung als Schmutzfänger in Waschlaugen oder dergleichen.

2. Faservlies, insbesondere nach Anspruch 1, dadurch gekennzeichnet, daß die Fasern (2) Kunststoffasern sind.

3. Faservlies, insbesondere nach einem oder mehreren der vorhergehenden Ansprüche, gekennzeichnet durch eine Kombination von aus unterschiedlichen Materialien bestehenden Fasern (2).

4. Faservlies, insbesondere nach einem oder mehreren der vorhergehenden Ansprüch, gekennzeichnet durch eine Kombination von aus unterschiedlichen Materialien bestehenden Kunststoffasern.

5. Faservlies, insbesondere nach einem oder mehreren der vorhergehenden Ansprüche, gekennzeichnet durch Polypropylenfasern und/oder Polyamidfasern und/oder kationisierte Zellulosefasern.

6. Faservlies, insbesondere nach einem oder mehreren der vorhergehenden Ansprüche, dadurch gekennzeichnet, daß das Faservlies (1) einseitig eine flüssigkeitsundurchläsige Folie (3) aufweist.

7. Verwendung eines Faservlieses, insbesondere nach einem oder mehreren der vorhergehenden Ansprüche, als Schmutzfänger in Waschlaugen oder dergleichen.

8. Verwendung eines einseitig mit Folie versehenen Faservlieses, insbesondere nach einem oder mehreren der vorhergehenden Ansprüche, zum vor dem Waschprozeß erfolgenden Detachieren von Verschmutzungen an Textilien.

9. Verwendung eines einseitig mit Folie versehenen Faservlieses, insbesondere nach einem oder mehreren der vorhergehenden Ansprüche, zum vor dem Waschprozeß erfolgenden Dosieren von flüssigem Waschmittel, wobei das Waschmittel vom saugfähigen bzw. flüssigkeitspeichernden Faservlies aufgenommen ist und das getränkte Faservlies zusammen mit den Wäschestükken in die Trommel einer Waschmaschine oder dergleichen gegeben wird.

10. Verfahren zum Waschen von Wäsche in einer Waschlauge oder dergleichen, dadurch gekennzeichnet, daß ein Faservlies gemäß einem oder mehreren der vorhergehenden Ansprüche als Schmutzfänger in die Waschlauge eingebracht wird.

# FIG. 1

# FIG.2